# EUROPÄISCHE PATENTSCHRIFT

(11) **EP 0 589 182 B1**
(45) Veröffentlichungstag und Bekanntmachung des Hinweises auf die Patenterteilung: **27.08.1997**
(21) Anmeldenummer: 93112202.2
(22) Anmeldetag: 29.07.1993
(51) Int. Cl.: A61N 1/05

(54) **Anordnung zur Explantation einer Elektrodenvorrichtung**
Arrangement for explantation of an electrode device
Arrangement pour l'explantation d'un dispositif d'électrodes

(30) Priorität: 23.09.1992 SE 9202746
(43) Veröffentlichungstag der Anmeldung: 30.03.1994
(73) Patentinhaber: Pacesetter AB, 171 95 Solna (SE)
(72) Erfinder: Nyman, Per, S-182 64 Djursholm (SE); Lindgren, Anders, S-183 40 Täby (SE)
(74) Vertreter: Lettström, Richard Wilhelm

(56) Entgegenhaltungen:
- EP-A- 0 368 568
- WO-A-91/19532
- US-A- 4 574 800

## Beschreibung

Die Erfindung betrifft eine Anordnung zur Explantation einer Elektrodenvorrichtung zur intrakorporalen Stimulation von Körpergewebe mit einem Elektrodenkabel, das einen langgestreckten, flexiblen wendelförmigen Leiter umfasst, dessen Aussenseite mit einer Isolierschicht versehen ist und dessen Innenseite einen Kanal bildet und dessen distales Ende mit einem Elektrodenkopf verbunden ist, wobei die Explantationsanordnung, die in den Kanal einschiebbar ist, aus einem mandrinförmigen Organ besteht, das mit Hilfe von Mitteln, die am distalen Ende des Organs wirken, im Kanal befestigbar sind.

Eine in einem Patienten permanent implantierte Elektrodenvorrichtung, z.B. eine Herzschrittmacherelektrode, wird nach einiger Zeit von Bindegewebe umschlossen, das das Elektrodenkabel derart verankert, dass es schwer ist, das Kabel von der Herzwand zu lösen. Es kann sogar gefährlich sein, an dem freigelegten proximalen Ende des Elektrodenkabels zu ziehen, da gesundes Gewebe im Bereich der Verankerungsstelle losgerissen oder auf andere Weise zerstört werden kann. Daher wählt der Arzt häufig die Möglichkeit, das Elektrodenkabel drin zu lassen und dieses durch eine weitere Elektrodenvorrichtung zu ersetzen. In bestimmten Fällen, wenn z.B. mehrere ausgediente, abgeschnittene Elektrodenkabel im Herz appliziert sind oder wenn eine Entzündung entsteht oder das Risiko, dass die Elektrodenkabel eine Vene oder die Herzwand penetrieren, vorhanden ist, muss oder müssen wenigstens ein oder mehrere Elektrodenkabel entweder durch einen operativen Eingriff oder mit Hilfe einer Explantationsanordnung entfernt werden.

Eine Explantationsanordnung der eingangs genannten Art ist durch den Artikel in der Fachzeitschrift "European Journal of Cardiac Pacing and Electrophysiology" Band 2, Nr. 2, Seite A 77 vom Juni 1992 mit dem Titel "Removal of Chronic implanted leads using a new technology in 25 cases" von Reinhardt Höcherl and Babczynski bekannt. Das mandrinförmige Organ ist an seinem distalen Ende mit nach hinten gerichteten Widerhaken versehen. Beim Einführen des Organs in den Kanal liegen die Widerhaken mehr oder weniger dicht an dem Organ. Wenn das Organ bzw. die Widerhaken am Platz ist bzw. sind, richten sich die Widerhaken derart auf, dass sie gegen die Innenwand des Kanals zu liegen kommen. Bei einer Explantation des Elektrodenkabels zieht der Arzt am Organ, wobei die Widerhaken in den wendelförmigen Leiter, der die Innenwand des Kanals bildet, eingreifen. Ein Nachteil der beschriebenen Explantationsanordnung ist, dass lediglich die kleinen freien Stirnseiten der Widerhaken in die Innenwand eingreifen. Dies kann zu einer Deformation wie z.B. einer Ausdehnung der Wendelform des Leiters führen, so dass die Widerhaken wenigstens zum Teil den Griff verlieren. Ein weiterer Nachteil ist, dass die Explantationsanordnung aufgrund der Widerhaken nicht umpositioniert oder aus dem Kanal herausgezogen werden kann. Eine solche Explantationsvorrichtung ist auch aus der WO91/19532 bekannt.

Der Erfindung liegt die Aufgabe zugrunde, eine Anordnung der eingangs genannten Art zur Explantation einer Elektrodenvorrichtung zu schaffen, bei der die Explantationsanordnung einfach und sicher im Kanal eines Elektrodenkabels befestigt werden kann gleichzeitig damit, dass die Anordnung bei einer bestimmten Zugbelastung vom Elektrodenkabel loslässt.

Diese Aufgabe ist erfindungsgemäss dadurch gelöst, dass die Mittel aus einem adhärenten Material bestehen. Abhängig von der Zusammensetzung des adhärenten Materiales kann das mandrinförmige Organ einer grösseren oder kleineren Belastung ausgesetzt werden, bevor es sich vom Elektrodenkabel löst. Dies ist von Vorteil, da der Arzt in der Regel im voraus weiss, in welcher Verfassung das Herz des Patienten ist. Durch die Verwendung eines adhärenten Materials kann das mandrinförmige Organ mit der proximalen Endseite des Elektrodenkopfes im Kanal der Elektrode verbunden werden. Hierdurch ist das Risiko, dass das Elektrodenkabel bei einer Zugbelastung deformiert wird, eliminiert worden. In einer solchen Haftlage kann auch der Elektrodenkopf mit dem Elektrodenkabel um seine Längsachse gedreht werden, um auf diese Weise die Elektrodenvorrichtung vom Herzgewebe zu lösen. Somit kann die kritischen Zugbelastungen am Herzgewebe verringert werden. Auch in dem Fall, wenn das Organ nicht mit dem Elektrodenkopf verbunden ist, kann das adhärente Material eine Anzahl Wicklungen am wendelförmigen Leiter im Elektrodenkabel verbinden, wodurch bei einer Zugbelastung eine Deformation des Leiters im Befestigungsbereich des Organs vermieden werden kann. Das adhäsive Material kann vorzugsweise aus einem schnellhärtenden und gewebefreundlichen Kleber wie z.B. Silikon- oder Zyanoakrylat bestehen. Weitere Beispiele an adhärenten Materialien sind reibungserhöhende Flüssigkeiten, ein festwerdendes Wachs, ein koagulierender Stoff oder eine phasenveränderbare chemische Komponente.

In einer vorteilhafen Weiterbildung der Erfindung wird vorgeschlagen, dass das adhärente Material in einer unter Druck zerplatzbaren Kapsel gespeichert ist. Die Kapsel, die in den Kanal eingeführt wird, kann nun mit Hilfe des mandrinförmigen Organs punktiert oder zerdrückt werden. Die Kapsel, die abhängig von der Grösse eine grössere oder kleinere Menge adhärentes Material beinhalten kann, kann auch an der distalen Endseite des mandrinförmigen Organs befestigt sein.

Im Hinblick auf eine einfache Ausgestaltung der Erfindung empfiehlt es sich, dass das mandrinförmige Organ rohrförmig ausgebildet ist, wobei mindestens das distale Ende des Kanals, der durch die Rohrform gebildet wird, mit dem adhärenten Material gefüllt ist. Hierdurch ist die Möglichkeit gegeben, eine verhältnismässig grosse Menge von dem adhärenten Material zu speichern.

In einer Weiterentwicklung dieser Ausführungsform der Erfindung wird vorgeschlagen, dass an dem proximalen Ende des mandrinförmigen Organs eine Anordnung angeschlossen ist, mit der das adhärente Material aus dem Kanal des Organs herausdrückbar ist. Durch diese Anordnung kann der Arzt so viel adhärentes Material herausdrücken, wie für ein gutes Anhaften des Organs notwendig ist. Ausserdem ist die Möglichkeit zu einer Positionierung des mandrinförmigen Organs an einer gewünschten Stelle im Kanal des Elektrodenkabels gegeben, da das adhärente Material unabhängig von der Lage des Organs aus dem Kanal des Organs herausgedrückt werden kann. Das Organ kann, wenn es nach einer bestimmten Zugbelastung von einer Befestigungsstelle gelöst wird, auch umpositioniert werden, indem der Arzt an der neu eingestellten Lage des Organs mehr adhärentes Material aus dem Kanal des Organs herausdrückt.

Nach der Erfindung wird ferner vorgeschlagen, dass das mandrinförmige Organ wenigstens an dessen distalem Ende mit einer Oberfläche versehen ist, die im Vergleich zur übrigen Oberfläche das Haftvermögen des adhärenten Materials erhöht. Eine solche Oberfläche kann z.B. eine poröse oder eine rillenförmige Struktur aufweisen.

Die Erfindung ist nachfolgend anhand mehrerer in den Zeichnungen dargestellten Ausführungsbeispielen näher erläutert. Es zeigen:

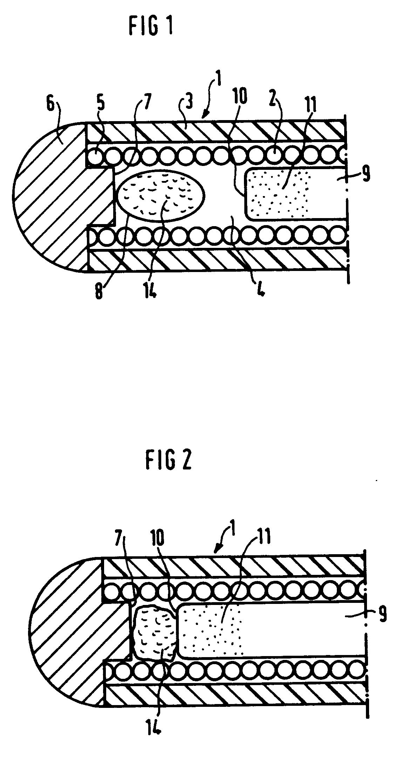

- FIG 1 bis 4: Seitenansichten von Elektrodenvorrichtungen im Längsschnitt mit in diesen Elektrodenvorrichtungen angebrachten Explantationsanordnungen nach der Erfindung in verschiedenen Lagen und Ausführungsformen.

In der FIG. 1 ist das distale Teil einer Elektrodenvorrichtung zur intrakorporalen Stimulation von Körpergeweben abgebildet. Die Elektrodenvorrichtung besteht aus einem Elektrodenkabel 1 das einen langgestreckten, flexiblen, wendelförmigen Leiter 2 umfasst, dessen Aussenseite mit einer Isolierschicht 3 und dessen Innenseite einen Kanal 4 bildet. An das distale Ende 5 des Leiters 2 ist ein Elektrodenkopf 6 für die Stimulation des Herzgewebes eines Patienten angeschlossen. In dem Kanal 4 ist gegen die Endseite 7 des Elektrodenkopfes eine beim Drücken zerplatzbare Kapsel 8 appliziert, die ein adhärentes Material 14 wie z.B. Zyanoakrylat beinhaltet. In dem Kanal 4 ist auch ein mandrinförmiges Organ 9 eingeführt. Die Kapsel 8 kann auch vorzugsweise an der distalen Endseite 10 des Organs befestigt sein. Das Organ 9 und die Kapsel 8 stellt hier eine Explantationsanordnung dar. Bei einer Explantation des Elektrodenkabels 1 wird das Organ 9 ein weiteres Stück in den Kanal 4 hineingedrückt, so dass die Endseite 10 des Organs auf die Kapsel 8 drückt, bis sie zerplatzt, wobei das adhärente Material 14 freigemacht wird und das mandrinförmige Organ 9, wie es in der FIG. 2 gezeigt ist, mit der Endseite 7 des Elektrodenkopfes verbindet. Dadurch, dass der Arzt mit Hilfe des Organs 9 das Elektrodenkabel im Bereich dessen Spitze fassen kann und damit diesen Teil für eine Zugbelastung aussetzt, ist das Risiko bezüglich einer Deformation des Leiters 2 sehr klein. Ausserdem ist es möglich, in dieser Lage das Organ 9 um seine Längsachse zu drehen, was dazu führt, dass auch das Elektrodenkabel 1 mit dem Elektrodenkopf 6 mitgedreht wird. Somit kann sich das Elektrodenkabel 1 vom Herzgewebe lösen. Auf diese Weise können sog. Zugbelastungen am Herzgewebe verringert werden, die sonst beim Herausziehen des Elektrodenkabels 1 eine unerwünschte Deformation oder Beschädigung des Herzmuskels verursachen können.

Das mandrinförmige Organ 9 ist an seinem distalen Ende mit einer Oberfläche 11 versehen, die im Vergleich zur übrigen Oberfläche das Haftvermögen des adhärenten Materials 14 erhöht. Die Oberfläche 11 kann vorzugsweise eine poröse oder eine rillenförmige Struktur aufweisen. Das Organ 9 kann nun, abhängig von der Zusammensetzung des adhärenten Materials 4, bei einer vorbestimmten Zugbelastung die Verbindung mit dem Elektrodenkabel 1 verlieren, bevor das Herzgewebe beschädigt wird.

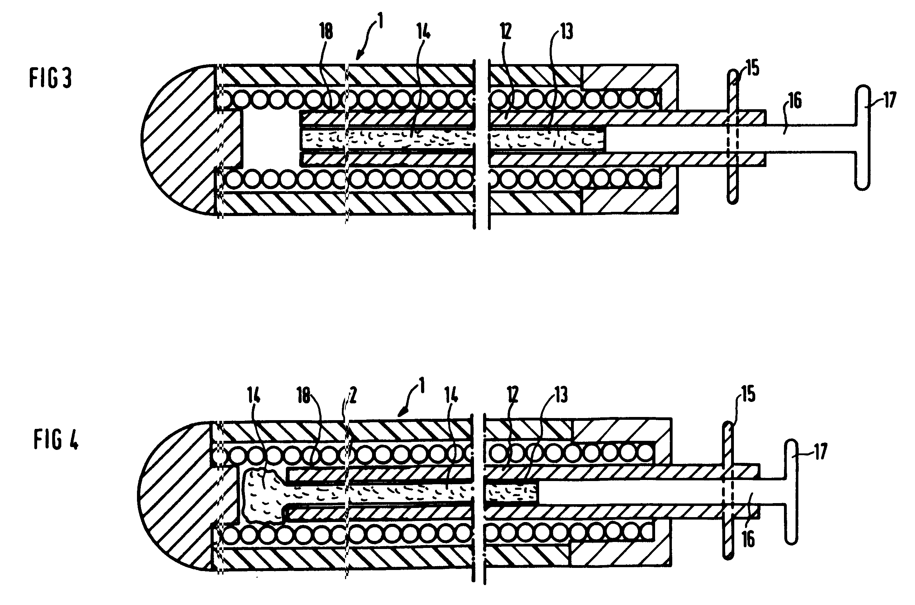

In der FIG. 3 ist der distale und der proximale Teil der Elektrodenvorrichtung nach den FIG. 1 und 2 gezeigt. In den Kanal 4 des Elektrodenkabels 1 ist eine Explantationsanordnung eingeführt, die in einem Längsschnitt gezeigt ist und die sich von der Explantationsanordnung, die in Verbindung mit den FIG. 1 und 2 gezeigt und beschrieben ist, unterscheidet. Diese Explantationsanordnung besteht aus einem rohrförmigen mandrinähnlichen Organ 12. Der Kanal 13, der durch die Rohrform gebildet ist, ist teilweise mit dem adhärenten Material 14 gefüllt. An das proximale Ende des Organs 12 ist ein Griff 15 angebracht. Im Kanal 13 des Organs 12 ist ferner eine verschiebbare Nadel 16, dessen freies Ende mit einer Platte 17 versehen ist, derart angeordnet, dass dessen Aussenmantelung gegen die Wand des Kanals 13 gleitet.

Bei einer Explantation des Elektrodenkabels 1 drückt der Arzt, wie es in der FIG. 4 dargestellt ist, das adhärente Material 14 aus dem Kanal 13 des Organs 12 heraus, indem die Nadel 16 mit Hilfe des Griffes und der Platte 17 weiter in den Kanal 13 eingeschoben wird. Durch das adhärente Material wird nun eine feste Verbindung zwischen dem Organ 12 und z.B. dem Elektrodenkopf 6 erhalten, wobei das Elektrodenkabel 1 in beschriebener Weise vom Herzgewebe losgezogen bzw. losgedreht werden kann. Auch dieses Organ ist mit einer Oberfläche 18 versehen, die das Haftvermögen des adhärenten Materials erhöht.

Die in den FIG 3 und 4 gezeigte Explantationsanordnung kann in einer gewünschten Lage im Kanal 4 des Elektrodenkabels positioniert werden. Die Positionierung erfolgt, indem das adhärente Material in beschriebener Weise an einer geeigneten Stelle im Kanal herausgedrückt wird und das Organ 12 mit dem Leiter 2 verbindet. Das adhärente Material 14 kann auch eine Anzahl Wicklungen der Wendel, die durch den Leiter 2 gebildet ist, verbinden, was bei einer Zugbelastung eine Deformation des Leiters 2 im Befestigungsbereich des Organs 12 vermeiden kann. Auch eine Umpositionierung dieser Explantationsanordnung kann erfolgen, indem das Organ 12 aus einer Befestigungsstelle losgezogen wird und adhärentes Material in einer weiteren Lage aus dem Kanal 13 des Organs herausgedrückt wird.

### Bezugszeichenliste

- 1: Elektrodenkabel
- 2: Leiter
- 3: Isolierschicht
- 4, 13: Kanal
- 5: das distale Ende des Leiters
- 6: Elektrodenkopf
- 7, 10: Endseite
- 8: Kapsel
- 9, 12: mandrinförmiges Organ
- 11, 18: Oberfläche
- 14: adhärentes Material
- 15: Griff
- 16: Nadel
- 17: Platte

## Patentansprüche

1. Anordnung zur Explantation einer Elektrodenvorrichtung zur intrakorporalen Stimulation von Körpergewebe mit einem Elektrodenkabel (1), das einen langgestreckten, flexiblen wendelförmigen Leiter (2) umfasst, dessen Aussenseite mit einer Isolierschicht (3) versehen ist und dessen Innenseite einen Kanal (4) bildet und dessen distales Ende mit einem Elektrodenkopf (6) verbunden ist, wobei die Explantationsanordnung, die in den Kanal einführbar ist, aus einem mandrinförmigen Organ (9) besteht, das mit Hilfe von Mitteln, die am distalen Ende des Organs wirken, im Kanal befestigbar sind, **dadurch gekennzeichnet**, dass die Mittel aus einem adhärenten Material (14) bestehen.

2. Anordnung nach Anspruch 1, **dadurch gekennzeichnet**, dass das adhärente Material (14) aus einem schnellhärtenden und gewebefreundlichen Kleber, einer reibungserhöhenden Flüssigkeit, einem festwerdenden Wachs, einem koagulierenden Stoff oder aus einer phasenveränderbaren chemischen Komponente besteht.

3. Anordnung nach Anspruch 1 oder 2, **dadurch gekennzeichnet**, dass das adhärente Material (14) in einer unter Druck zerplatzbaren Kapsel gespeichert ist.

4. Anordnung nach den Ansrpüchen 1 oder 2, **dadurch gekennzeichnet**, dass das mandrinförmige Organ (12) rohrförmig ausgebildet ist, wobei mindestens das distale Ende des Kanals (13), der durch die Rohrform gebildet wird, mit dem adhärenten Material (14) gefüllt ist.

5. Anordnung nach Anspruch 4, **dadurch gekennzeichnet**, dass an dem proximalen Ende des mandrinförmigen Organs (12) eine Anordnung (16,17) angeschlossen ist, mit der das adhärente Material (14) aus dem Kanal (13) des Organs (12) herausdrückbar ist.

6. Anordnung nach einem der Ansprüche 1 bis 5, **dadurch gekennzeichnet**, dass das mandrinförmige Organ (9,12) wenigstens an seinem distalen Ende mit einer Oberfläche (11,18) versehen ist, die im Vergleich zur übrigen Oberfläche das Haftvermögen des adhärenten Materiales (14) erhöht.

## Claims

1. Arrangement for the explantation of an electrode device for the intracorporal stimulation of body tissue, the electrode device having an electrode cable (1) which comprises an elongated, flexible, helical conductor (2) whose outside is provided with an insulating layer (3) and whose inside forms a duct (4) and whose distal end is connected to an electrode head (6), wherein the explantation arrangement, which can be inserted in the duct, consists of a mandrin-shaped element (9) which can be secured in the duct with the aid of means which act at the distal end of the element, **characterised in that** the means consist of an adherent material (14).

2. Arrangement according to claim 1, characterised in that the adherent material (14) consists of a rapid-hardening and tissue-friendly adhesive, a friction-increasing fluid, a solidifying wax, a coagulating substance or a phase-changeable chemical constituent.

3. Arrangement according to claim 1 or 2, characterised in that the adherent material (14) is stored in a capsule which can be burst under pressure.

4. Arrangement according to claim 1 or 2, characterised in that the mandrin-shaped element (12) is formed with a tubular shape, wherein at least the distal end of the duct (13) that is formed by the tubular shape, is filled with the adherent material (14).

5. Arrangement according to claim 4, characterised in that, at the proximal end of the mandrin-shaped element (12), there is attached an arrangement (16, 17) with which the adherent material (14) can be forced out of the duct (13) of the element (12).

6. Arrangement according to one of claims 1 to 5, characterised in that the mandrin-shaped element (9, 12) is provided, at least at its distal end, with a surface (11, 18) which increases the adhesive power of the adherent material (14) in comparison to the remaining surface.

## Revendications

1. Dispositif d'explantation d'un dispositif à électrode de simultation intracorporelle de tissus corporels comportant un câble (1) d'électrode, lequel comprend un conducteur (2) hélicoïdal souple et oblong, dont le côté extérieur est muni d'une couche (3) isolante, dont le côté intérieur forme un canal (4) et dont l'extrémité distale est reliée à une tête (6) d'électrode, le dispositif d'explantation, qui peut être introduit dans le canal, étant constitué d'un organe (9) en forme de mandrin, qui peut être fixé dans le canal à l'aide de moyens, qui agissent, à l'extrémité distale de l'organe, caractérisé en ce que les moyens sont en un matériau (14) adhérent.

2. Dispositif suivant la revendication 1, caractérisé en ce que le matériau adhérent (14) est constitué d'une colle durcissant rapidement et compatible avec les tissus, d'un liquide augmentant les frottements, d'une cire se solidifiant , d'une substance coagulante ou d'un composé chimique pouvant changer de phase.

3. Dispositif suivant la revendication 1 ou 2, caractérisé en ce que le matériau adhérent (14) est stocké dans une capsule pouvant crever sous pression.

4. Dispositif suivant la revendication 1 ou 2, caractérisé en ce que l'organe (12) en forme de mandrin est tubulaire, au moins l'extrémité distale du canal (13), qui est formé par la forme du tube, étant remplie par le matériau adhérent (14).

5. Dispositif suivant la revendication 4, caractérisé en ce qu'il est connecté à l'extrémité proximale de l'organe (12) en forme de mandrin un dispositif (16,17), par lequel le matériau adhérent (14) peut être sorti du canal (13) de l'organe (12).

6. Dispositif suivant l'une des revendications 1 à 5, caractérisé en ce que l'organe (9,12) en forme de mandrin est muni, au moins à son extrémité distale, d'une surface (11, 18), qui augmente le pouvoir d'adhérence du matériau adhérent (14) par rapport au reste de la surface.
